# FASCICULE DE BREVET EUROPEEN

(11) **EP 3 108 250 B1**
(45) Date de publication et mention de la délivrance du brevet: **22.05.2019**
(21) Numéro de dépôt: 15706913.9
(22) Date de dépôt: 12.02.2015
(51) Int. Cl.: G01N 33/569

(54) **PROCÉDÉ ET KIT POUR DÉTERMINER LA PROBABILITÉ POUR UN PATIENT D'ÉVOLUER VERS UNE DENGUE SÉVÈRE**
VERFAHREN UND KIT ZUR BESTIMMUNG DER WAHRSCHEINLICHKEIT EINES PATIENTEN, EINEN SCHWEREN FALL VON DENGUE ZU ENTWICKELN
METHOD AND KIT FOR DETERMINING THE PROBABILITY THAT A PATIENT WILL DEVELOP A SEVERE CASE OF DENGUE

(30) Priorité: 18.02.2014 FR 1451274
(43) Date de publication de la demande: 28.12.2016
(73) Titulaire: Biomérieux, 69280 Marcy l'Étoile (FR)
(72) Inventeur: BEDIN, Frédéric, 69007 Lyon (FR); FRAGNOUD, Romain, 91260 Juvisy sur Orge (FR)
(74) Mandataire: Bitaud, Valérie Marie-Odile
(86) Numéro de dépôt international: PCT/FR2015/050352
(87) Numéro de publication internationale: WO 2015/124850

(56) Documents cités:
- WO-A1-2013/110894
- WO-A1-2013/148335
- US-A1- 2010 068 147
- MANABU MAEDA ET AL: "Plasma levels of molecular markers of blood coagulation and fibrinolysis in progressive systemic sclerosis (PSS)", JOURNAL OF DERMATOLOGICAL SCIENCE, vol. 11, no. 3, 1 mars 1996 (1996-03-01), pages 223-227, XP055161216, ISSN: 0923-1811, DOI: 10.1016/0923-1811(95)00445-9
- SRICHAIKUL T ET AL: "Platelet function during the acute phase of dengue hemorrhagic fever", SOUTHEAST ASIAN JOURNAL OF TROPICAL MEDICINE AND PUBLIC HEALTH, PROJECT OD SEAMEO, BANGKOK, TH, vol. 20, no. 1, 1 mars 1989 (1989-03-01), pages 19-25, XP008174065, ISSN: 0125-1562
- ONG S P ET AL: "Identifying Host Factors Involved in Mediating Vascular Permeability During Dengue Virus Infection", INTERNATIONAL JOURNAL OF INFECTIOUS DISEASES, INTERNATIONAL SOCIETY FOR INFECTIOUS DISEASES, HAMILTON, CA, vol. 12, 1 décembre 2008 (2008-12-01), page e297, XP026612869, ISSN: 1201-9712, DOI: 10.1016/J.IJID.2008.05.797 [extrait le 2008-12-01]

## Description

La présente invention a pour objet un procédé pour le pronostic précoce d'une dengue sévère ou dengue hémorragique utilisant des marqueurs protéiques tel que défini dans les revendications.

Ces 30 dernières années, la dengue, une maladie virale transmise par les moustiques hématophages urbains du genre Aedes, s'est répandue à travers le monde de manière inquiétante. C'est actuellement un véritable problème de santé publique pour plus d'une centaine de pays situés dans la zone subtropicale, particulièrement dans les zones pacifique-ouest, Amérique du sud et Asie du sud-est. L'émergence de la maladie est due en grande partie à l'explosion démographique et à l'urbanisation anarchique. Les anomalies climatiques ont également un rôle non négligeable.

A ce titre, la dengue pourrait émerger dans les régions occidentales du globe jusqu'alors épargnées par le virus. Ainsi, *Aedes albopictus*, un des vecteurs de la maladie, a récemment été trouvé dans le nord de l'Italie et dans le sud de la France. Dernièrement, des cas de dengue autochtones ont été signalés dans le sud de la France. On estime que près de 3 milliards de personnes sont exposés aux risques de dengue. Près d'un million d'hospitalisations sont recensées annuellement et les décès se comptent par milliers. Les enfants sont les principales victimes de la maladie.

Le virus de la dengue est un virus enveloppé à ARN monocaténaire de polarité positive de la famille des Flaviviridae. Le génome du virus (11 000 nucléotides) code pour une polyprotéine d'environ 3400 acides aminés qui subit un clivage co- et post-traductionnel qui résulte en des protéines structurales (C, prM, E) et des protéines non-structurales (NS1, NS2A, NS2B, NS3, NS4A, NS4B, NS5). Il existe 4 sérotypes viraux (DV1 à DV4), qui peuvent coexister en zones d'endémie. Il y a environ 70% d'homologie de séquences entre les différents sérotypes. L'infection par un sérotype donné confère une immunité sur le long terme pour ce sérotype. La protection croisée ne dure que quelques mois : la réinfection est donc possible avec un sérotype différent.

L'infestation débute avec la piqûre du moustique infecté par un des virus de la dengue. L'incubation, période lors de laquelle le virus se réplique dans le sang sans pour autant donner de symptôme, dure généralement de 4 à 10 jours. Les premiers signes surviennent après la période d'incubation.

Dans sa forme classique (fièvre de dengue « classique » ou *Dengue Fever* : DF), la dengue se caractérise par une hyperthermie d'apparition brutale accompagnée d'un ou plusieurs des symptômes suivants : frissons, céphalées, douleurs articulaires et/ou musculaires, nausées, vomissements. Une éruption cutanée peut également survenir, généralement vers le 5eme jour des symptômes. Cette phase fébrile aiguë, qui correspond à la phase virémique, dure généralement de 3 à 5 jours (extrêmes : 2 à 7 jours). Plus de 95% des cas ne présenteront aucun signe de gravité et guériront sans complication en moins de 7 jours.

Dans 2 à 4% des cas, le patient peut développer une phase critique caractérisée par un syndrome de fuite plasmatique plus ou moins sévère et une élévation de l'hématocrite conduisant à une dengue hémorragique (*Dengue Hemorrhagic Fever* : DHF). Cette phase apparaît typiquement (mais pas obligatoirement) au moment de la défervescence thermique, autour du 4 ou 5ème jour. Elle est généralement brève (24 à 48h) mais peut évoluer vers une forme sévère caractérisée par des manifestations hémorragiques majeures, un état de choc et/ou la défaillance d'un ou plusieurs organes. Le plus souvent, l'évolution vers une forme sévère est annoncée par un (des) signe(s) d'alerte, tels que :
- de la fièvre (température supérieure à 39°C) après le 5ème jour ;
- des douleurs abdominales intenses, diarrhées persistantes, vomissements incoercibles avec refus total d'alimentation ;
- des oedèmes et/ou épanchement mineur ;
- des saignements des muqueuses ne cédant pas spontanément ;
- une agitation ou léthargie prononcée ;
- une thrombopénie ;
- des signes d'hémoconcentration.

Dans les cas les plus sévères, la fuite de plasma peut entraîner un choc hypovolumique mortel (*Dengue Shock Syndrome* : DSS) si le patient n'est pas pris en charge rapidement. Des atteintes hépatiques
et neurologiques, rares mais mortelles, sont également associées à la sévérité de la maladie. Variable selon les épidémies, le taux de mortalité peut atteindre 5% des cas déclarés de DHF. Ce taux peut augmenter jusqu'à 20% sans une prise en charge hospitalière ou des traitements adaptés.

Par simplification, ces cas sévères seront dénommés dans la suite de la description, par opposition aux dengues classiques DF, dengues sévères DS.

90% des cas de dengues sévères ont lieu lors d'une infection secondaire par un sérotype hétérologue et 10% lors d'une infection primaire, habituellement chez des nourrissons âgés de 6 mois à 1 an. Il existe plusieurs facteurs qui influent sur la sévérité de l'infection, tels que les facteurs de l'hôte, le sérotype et le génotype du virus, l'ordre et l'intervalle de succession des virus infectants, la qualité et la quantité d'anticorps à réactions croisées et la réponse CD4/CD8. Des études ont montré une corrélation entre la charge virale et la sévérité de la maladie. Cependant, les causes exactes de l'apparition d'une dengue sévère ne sont toujours pas connues. Jusqu'à présent, aucun déterminant spécifique de la virulence n'a été mis en évidence. De plus, comme il n'existe pas de vaccins contre le virus de la dengue, les seuls traitements disponibles sont des traitements symptomatiques. C'est pourquoi, il est important de pouvoir surveiller les épidémies et de pronostiquer les cas sévères pour une prise en charge hospitalière adaptée.

Srichaikul et *al*. [7], dans leurs travaux, ont décrit l'augmentation du facteur plaquettaire 4 durant la phase aigüe de la dengue hémoragique par rapport aux patients ayant une dengue classique. Néanmoins aucune caractéristique prédictive n'est décrite.

Les méthodes actuellement utilisées pour le diagnostic de la dengue ne permettent pas de pronostiquer une évolution vers une dengue sévère. Tout au plus, les méthodes sérologiques permettent de discriminer infections primaires et secondaires et les méthodes moléculaires permettent de détecter le virus et d'effectuer le sérotypage[1, 2, 3, 4].

La présente invention répond aux problématiques présentées ci-dessus par un procédé qui permet à la fois une détection précoce et spécifique de protéines dans un échantillon sanguin permettant de pronostiquer les patients évoluant vers des dengues sévères. En effet, les inventeurs ont trouvé de manière surprenante que des protéines de l'hôte étaient exprimées de manière plus ou moins abondante (surexprimées/sous-exprimées) dans les cas de patients évoluant vers des dengues sévères en comparaison avec leur quantité ou expression dans des cas de patients restant avec des dengues classiques (c'est-à-dire n'évoluant pas vers des dengues sévères) dans des échantillons sanguins constitués par exemple par le plasma. Tout particulièrement, ils ont montré pour la première fois et de manière totalement inattendue que le facteur plaquettaire 4 (ou platelet factor 4 ou PF4) est sous exprimé dans le cas de patients évoluant vers des dengues sévères et constitue donc un marqueur pronostique de la dengue sévère.

La demande WO 2013/148335 décrit un procédé permettant de déterminer si un patient est atteint d'une infection par le virus de la dengue susceptible d'évoluer vers une dengue sévère par la détection dans l'échantillon du patient de l'expression d'au moins deux protéines choisies parmi le facteur de croissance de l'endothélium vasculaire, la chymase-1 et l'α2-MG. Néanmoins, ce document ne décrit pas le marqueur PF4 et encore moins une sous-expression de la quantité sanguine de ce marqueur chez les patients qui vont évoluer vers une dengue sévère.

Aussi la présente invention a pour objet un procédé pour pronostiquer, *in vitro,* dans un échantillon sanguin, la probabilité pour un patient d'évoluer vers une dengue sévère, dans lequel :
a)on détermine la quantité d'au moins un marqueur qui est le platelet factor 4 dans ledit échantillon sanguin,
b)on compare la quantité de platelet factor 4 déterminée dans l'étape a) avec une quantité de référence dudit marqueur obtenu à partir d'un groupe d'individus qui ont été diagnostiqués comme étant atteints d'une dengue non sévère, dans lequel si la quantité de platelet factor 4 déterminée dans l'étape a) est inférieure à la quantité de référence établie dans l'étape b), on pronostique que le patient va évoluer vers une dengue sévère.

Selon le procédé de l'invention on peut aussi déterminer dans l'étape a) la quantité d'au moins un autre marqueur choisi parmi l'olfactomédine 4 (OLFM4) et l'α2-macroglobuline (A2M) ou la quantité respective des deux marqueurs dans l'échantillon sanguin et dans l'étape b) on compare la quantité du marqueur ou des deux marqueurs de l'étape a) avec une quantité de référence obtenue à partir d'un groupe d'individus qui ont été diagnostiqués comme étant atteints d'une dengue non sévère et si la quantité de l'olfactomlédine-4 déterminée dans l'étape a) est supérieure à la quantité de référence établie dans l'étape b) et/ou la quantité d'α2-macroglobuline est inférieure à la quantité de référence établie dans l'étape b), on détermine que le patient va évoluer vers une dengue sévère.

L'invention concerne également l'utilisation du facteur plaquettaire 4 comme marqueur protéique pour déterminer, *in vitro,* dans un échantillon sanguin, la probabilité pour un patient d'évoluer vers une dengue sévère.

L'invention concerne également l'utilisation d'un kit pour déterminer, *in vitro,* la probabilité pour un patient d'évoluer vers une dengue sévère, le kit comprenant un partenaire de liaison du facteur plaquettaire 4 et un partenaire de liaison de la protéine NS1 du virus de la dengue, lesdits partenaires de liaison étant choisis parmi un récepteur, un anticorps, un fragment d'anticorps, un analogue d'anticorps ou tout autre ligand. L'invention concerne également un kit pour déterminer, *in vitro,* la probabilité pour un patient d'évoluer vers une dengue sévère comprenant un partenaire de liaison du facteur plaquettaire 4 et un partenaire de liaison de l'α2-macroglobuline, lesdits partenaires de liaison étant choisis parmi un récepteur, un anticorps, un fragment d'anticorps, un analogue d'anticorps ou tout autre ligand.

L'invention concerne également un kit pour le pronostic, *in vitro,* d'une dengue sévère comprenant un partenaire de liaison du facteur plaquettaire 4, un partenaire de liaison de l'olfactomedine 4, un partenaire de liaison de l'a2-macroglobuline et un partenaire de liaison de la protéine NS1 du virus de la dengue, lesdits partenaires de liaison étant choisis parmi un récepteur, un anticorps, un fragment d'anticorps, un analogue d'anticorps ou tout autre ligand.

### Définitions

Par échantillon sanguin on entend le sang total, le sérum et le plasma.

Par un groupe d'individus qui ont été diagnostiqués comme étant atteints d'une dengue non sévère, utilisé pour déterminer la quantité de référence du marqueur d'intérêt, on entend bien entendu que le groupe d'individu n'a pas évolué vers une dengue sévère. Aussi, la comparaison à l'étape b) de la quantité de platelet factor 4 déterminée dans l'étape a) se fait avec une quantité de référence dudit marqueur obtenu à partir d'un groupe d'individus atteints de dengue, mais n'ayant pas évolué vers une dengue sévère.

Par partenaire de liaison on entend par exemple les récepteurs, les anticorps, les fragments d'anticorps, les analogues d'anticorps et tout autre ligand capable de se lier à une protéine.

Les anticorps partenaires de liaison sont par exemple soit des anticorps polyclonaux, soit des anticorps monoclonaux.

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Par « analogues d'anticorps » on entend des composés biologiques et/ou chimiques qui possèdent les mêmes capacités de liaison que les anticorps ou fragments d'anticorps ou des capacités de liaison similaires. En particulier, les analogues d'anticorps incluent de petites protéines qui, comme les anticorps, sont capables de se lier à une cible biologique, permettant ainsi de la détecter, de la capturer ou tout simplement de la cibler au sein d'un organisme ou d'un échantillon biologique. Les champs d'application de ces analogues d'anticorps sont pratiquement aussi vastes que ceux des anticorps. A titre d'exemple, on peut citer les Nanofitines™ , petites protéines commercialisées par la société AFFILOGIC.

Les partenaires de liaison spécifiques de la protéine recherchée peuvent être utilisés comme réactif de capture, comme réactif de détection ou comme réactifs de capture et de détection.

La visualisation des réactions immunologiques, c'est-à-dire de la liaison protéine/partenaire de liaison, peut être effectuée par tout moyen de détection par l'intermédiaire d'un marquage, du partenaire de liaison.

Par marquage, on entend la fixation d'un réactif marqueur capable de générer un signal détectable, c'est-à-dire un composé, une substance ou une particule qui peut être détecté (e) par des moyens visuels, fluorescents, instrumentaux.

Une liste non limitative de ces réactifs marqueurs consiste en :
- les particules métalliques ou d'alliages, telles que les particules d'or colloïdal,
- les particules de polymère, telles que les particules de latex colorés,
- les particules magnétiques,
- les molécules fluorescentes,
- les molécules chimioluminescentes.
A titre d'exemple de tests immunologiques tels que définis ci-dessus, on peut citer les méthodes « sandwich » et de « compétition ».

### Figures :

La majorité des figures illustre la validation des résultats par un test ELISA quantitatif effectué sur des échantillons individuels de patients prélevés durant la phase fébrile aiguë de la maladie, avant défervescence. Les patients étant restés en dengue classique sont notés DF et les patients ayant évolué ensuite vers une dengue sévère sont notés DS. Dans tous les cas la lecture est effectuée à une densité optique (DO) de 450nm. Les résultats ont été obtenus sur des échantillons d'origine géographique différente : la Colombie et le Cambodge. Sur les graphes obtenus(Logiciel GraphPad Prism V4.03), la médiane calculée est représentée par un trait horizontal. La boîte illustre les valeurs englobant 50% des individus. Les valeurs maximum et minimum sont également illustrées. Les valeurs prises en compte correspondent à la moyenne de deux essais indépendants effectués en duplicate.
La figure 1 illustre la présence de virus dans les fractions purifiées à partir du plasma de patients mais pas dans le contrôle (C), mis en évidence avec un western-blot utilisant un anticorps monoclonal dirigé contre la protéine virale E.
La figure 2 illustre les résultats obtenus pour le dosage quantitatif par test ELISA du marqueur PF4 sur des échantillons de plasma provenant de patients colombiens.
La figure 3 illustre les résultats obtenus pour le dosage quantitatif par test ELISA du marqueur PF4 sur des échantillons de plasma provenant de patients cambodgiens.
La figure 4 illustre les résultats obtenus pour le dosage quantitatif par test ELISA du marqueur OLFM4 sur des échantillons de plasma provenant de patients colombiens.
La figure 5 illustre les résultats obtenus pour le dosage quantitatif par test ELISA du marqueur OLFM4 sur des échantillons de plasma provenant de patients cambodgiens.
La figure 6 illustre les résultats obtenus pour le dosage quantitatif par test ELISA du marqueur A2M sur des échantillons de plasma provenant de patients cambodgiens.

Les exemples suivants ne sont fournis qu'à titre illustratif.

### Exemple 1 : Caractérisation des échantillons

15 échantillons de plasma colombiens positifs pour la dengue ont été sélectionnés, parmi lesquels 8 sont issus de patients restés en dengue classique sans évoluer vers une dengue sévère (patients/échantillons appelés par la suite DF ou dengue classique), et 7 sont issus de patients ayant ensuite développé une dengue sévère (patients/échantillons appelés par la suite DS ou dengue sévère). Les différents plasmas ont été regroupés, constituant respectivement un pool de plasma dengue classique DF et un pool de plasma dengue sévère DS pour ceux ayant évolué vers une dengue sévère. Tous les plasmas ont été prélevés durant la phase fébrile aiguë de la maladie, avant la phase critique, sur des malades ayant une infection secondaire. Les sérotypes concernés étaient les sérotypes 1, 2 et 3. Tous les patients ayant développé une dengue sévère étaient hospitalisés et présentaient des signes d'hémorragies. Aucune comorbidité n'a été rapportée [5]. Tous les plasmas ont été contrôlés comme étant NS1-positifs (Kit Platelia Dengue Biorad) et la charge virale a également été vérifiée en Q-RTPCR par un kit commercial (PrimerDesign) suivant les instructions du fournisseur : le nombre moyen de copies d'ARN viral a été estimé à 4.10⁶ et 4.1 10⁷ pour les pools dengue classique (DF) et dengue sévère (DS), respectivement. Les pools constitués correspondent à un volume d'environ 2 mL de plasma. Avant purification, les mélanges de plasma ont été centrifugés 5 min à 1000g et à 4°C, afin d'enlever les impuretés présentes dans l'échantillon et d'obtenir des échantillons clarifiés.

Les critères de sélection des plasmas sont décrits dans le tableau 1 qui suit. Les prélèvements ont été effectués après apparition des symptômes.

**Tableau 1**

| Plasma colombiens | | POOL DF | POOL DS |
|---|---|---|---|
| General | Nombre | 8 | 7 |
| | Age (Moyenne) | 26.8 | 33.7 |
| | M/F ratio | 6/2 | 3/4 |
| | Jour | 3.3 | 2.7 |
| | Infection secondaire | Oui | Oui |
| | NS1 | Positif | Positif |
| Serotype | DV1 | 1 | 0 |
| | DV2 | 1 | 3 |
| | DV3 | 6 | 4 |

Ces pools d'échantillons de plasma ont ensuite été purifiés pour obtenir des fractions enrichies en virus comme décrit ci-dessous.

### Exemple 2 : Purification des échantillons

Toutes les étapes sont effectuées à 4°C. Les échantillons clarifiés sont complétés par 8 mL de PBS pH8 froid (PBS8), puis sont centrifugés 2h à 41 000 g dans une ultracentrifugeuse OptimaL90 (Beckman). Le rotor utilisé est le rotor SW41 (Beckman). Après centrifugation, le surnageant est enlevé et le culot viral obtenu est resuspendu dans 200 microlitres de PBS8 puis chargé sur un gradient discontinu constitué de 5 mL de saccharose 60% (p/p) en PBS8 et 5 mL de saccharose 20% (p/p) en PBS8. Après une nouvelle centrifugation de 2h à 41 000 g, un anneau enrichi en virions, localisé à l'interface entre les deux solutions de saccharose, est prélevé à la pipette, dilué 10 fois par du PBS8 et finalement centrifugé une dernière fois 2h à 41 000 g. Le culot obtenu est re-suspendu dans 200 microlitres de PBS8.

Cette re-suspension est ensuite purifiée par utilisation d'un polyelectrolyte insoluble, le Viraffinity (BioSupportGroup, USA). Pour cela, 200 microlitres d'un tampon MN (60mM MES pH6.5, 150 mM NaCl)sont ajoutés à la suspension virale ainsi que 100 microlitres de Viraffinity. Le mélange est incubé 5 min à température ambiante puis centrifugé 10 min à 1000g , selon les instructions du fournisseur. Le surnageant est enlevé et le culot de polymère est rincé 3 fois avec 200 microlitres de tampon MN. Les protéines virales sont récupérées par chauffage du polymère 5 min/70°C en présence de 50 microlitres d'un tampon contenant du SDS (Novex InVitrogen) puis d'une centrifugation de 5 min à 1000 g.

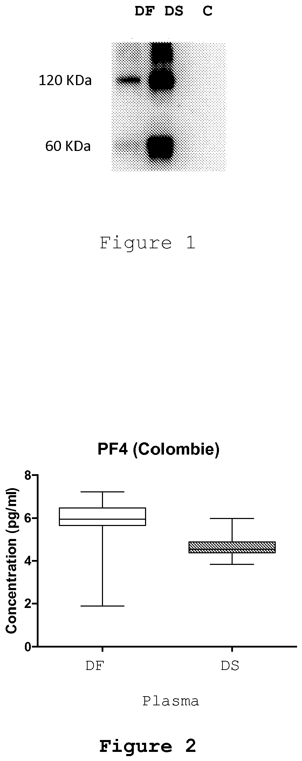

La présence du virus dans les échantillons finaux a été vérifiée par immunoblot avec un anticorps monoclonal dirigé contre la protéine d'enveloppe du virus de la dengue (protéine E). Comme illustré sur la figure 1 de forts signaux à 60 KDa et 120 KDa, correspondant respectivement aux formes monomériques et dimériques de la protéine d'enveloppe, sont spécifiquement détectés par l'anticorps monoclonal dans les pools de plasma . En revanche, la protéine d'enveloppe n'a pas été détectée sur un contrôle correspondant à un pool de plasmas sains (non dengue) purifié de la même façon que ce qui a été décrit ci-dessus.

### Exemple 3 : Identification des protéines spécifiques de chaque pool de plasma dengue classique DF et dengue sévère DS par spectrométrie de masse (MS)

### Méthode :

Les préparations virales et l'échantillon contrôle obtenus selon l'Exemple 2 sont déposées sur un gel de polyacrylamide non dénaturant, et mises à migrer jusqu'à ce que les protéines entrent à l'intérieur du gel, ceci afin de dessaler l'échantillon. La bande contenant les protéines est excisée manuellement puis lavée 3 fois dans un tampon contenant 50% d'acétonitrile pour finalement être séchée dans 100% d'acétonitrile. Le gel est ensuite réhydraté dans une solution à 7% d'H₂O₂ avant d'être lavé de nouveau. Une solution de trypsine diluée dans 25mM NH4HCO3 est ensuite ajoutée pour une hydrolyse à 37°C sur la nuit. Les peptides ainsi obtenus sont extraits par des extractions séquentielles de 15 minutes avec 30 microlitres d'acétonitrile 50%, 30 microlitres d'acide formique 5% et 30 microlitres d'acétonitrile 100%. Ces extractions séquentielles sont mélangées, séchées sous vide et resuspendues dans une solution contenant 5% d'acétonitrile et 0.1% d'acide trifluoroacétique. Après quantification des échantillons, une quantité définie de peptides est analysée par chromatographie nanoliquide couplée en tandem à de la spectrométrie de masse(Ultimate 3000, Dionex et LTQ-Orbitrap VelosPro, Thermo Fisher Scientific). Les résultats sont acquis grâce au logiciel Xcalibur (ThermoFisher) et transformés automatiquement par le logiciel Mascot Daemon V2.2 (Matrix Science). La recherche se fait ensuite sur les bases de données Swissprot et Trembl via Mascot2.2. Chaque expérience a été menée deux fois, de façon indépendante. L'identification des protéines a été réalisée par le laboratoire EDYP Service (CEA Grenoble, France).

### Résultats :

La protéine virale d'enveloppe E a été identifiée de façon répétée dans les échantillons contenant du virus. La séquence du peptide majoritairement identifié est GWGNGCGLLFKG. Ce résultat confirme la présence du virus dans la fraction purifiée.

Pour les protéines d'origine cellulaire, identifiées par protéomique sur les pools de plasmas purifiés, les résultats obtenus sont résumés dans les tableaux 2a et 2b. Dans ces tableaux, seules les protéines ayant une variance inférieure à 25% pour le nombre de peptides trouvés d'une expérience à l'autre ont été considérées. De même, pour l'échantillon dengue sévère, un nombre de peptide supérieur à 2 a été requis. Suivant ces critères, 189 protéines ont finalement été sélectionnées. Ces protéines sont décrites dans le tableau 2a et 2b qui suit. Un rapport « nombre de peptides échantillon dengue sévère (DS) »/ «nombre de peptides échantillon dengue classique (DF)» (DS/DF) a pu être calculé pour une majorité de ces protéines (cf. tableau 2a). Certaines protéines ont été identifiées uniquement dans l'échantillon DS (cf. tableau 2b), dans ce cas le rapport DS/DF n'était pas calculable.

**Tableau 2a**

| **Numéro d'accession** | **Nom protéine** | **Nombre moyen de peptides DF** | **Nombre moyen de peptides DS** | **Ratio peptides DS/DF** |
|---|---|---|---|---|
| P09871 | Complément C1s subcomponent (C1 esterase) | 3,5 | 14,5 | 4,14 |
| P07225 | Vitamin K-dependent protein S | 1 | 4 | 4,00 |
| P01008 | Antithrombin-III (ATIII) (Serpin C1) | 3 | 9,5 | 3,17 |
| Q16610 | Extracellular matrix protein 1 (Secretory component p85) | 1 | 3 | 3,00 |
| P27918 | Properdin (Complement factor P) | 1,5 | 4 | 2,67 |
| B4E1B2 | Serotransferrin | 11 | 29 | 2,64 |
| Q53H26 | Transferrin variant (Fragment) | 11 | 29 | 2,64 |
| B4DPQ0 | Complément C1r subcomponent | 5 | 13 | 2,60 |
| P01019 | Angiotensinogen (Serpin A8) | 1 | 2,5 | 2,50 |
| B4DDU2 | Tubulin alpha-ubiquitous chain | 2,5 | 6 | 2,40 |
| P01779 | Ig heavy chain V-III region TUR | 2,5 | 6 | 2,40 |
| P68366 | Tubulin alpha-4A chain (Alpha-tubulin 1) | 3 | 6,5 | 2,17 |
| P00734 | Prothrombin (Coagulation factor II) | 2 | 4 | 2,00 |
| P21333 | Filamin-A (Actin-binding protein 280) | 14,5 | 28,5 | 1,97 |
| P00450 | Ceruloplasmin (EC 1.16.3.1) (Ferroxidase) | 8,5 | 16,5 | 1,94 |
| E7EX29 | 14-3-3 protein zeta/delta (Fragment) | 4,5 | 8,5 | 1,89 |
| P63104 | 14-3-3 protein zeta/delta (KCIP-1) | 4,5 | 8,5 | 1,89 |
| B4E1D8 | cDNA FLJ51597, highly similar to C4b-binding protein | 10,5 | 19 | 1,81 |
| P18206 | Vinculin (Metavinculin) | 5 | 9 | 1,80 |
| P12814 | Alpha-actinin-1 (Alpha-actinin cytoskeletal isoform) | 6,5 | 11,5 | 1,77 |
| O43707 | Alpha-actinin-4 (F-actin cross-linking protein) | 6,5 | 11,5 | 1,77 |
| P06396 | Gelsolin (AGEL) (Actin-depolymerizing factor) | 3,5 | 6 | 1,71 |
| P05106 | Integrin beta-3 (Platelet membrane glycoprotein IIIa) (GPIIIa) | 5,5 | 9 | 1,64 |
| P08514 | Integrin alpha-IIb (GPIIb) (Platelet membrane glycoprotein IIb) | 12,5 | 20 | 1,60 |
| P01833 | Polymeric immunoglobulin receptor (PIgR) | 2,5 | 4 | 1,60 |
| Q9Y490 | Talin-1 | 28,5 | 43 | 1,51 |
| A2J1N9 | Rheumatoid factor RF-ET12 (Fragment) | 3 | 4,5 | 1,50 |
| Q5NV90 | V2-17 protein (Fragment) | 3 | 4,5 | 1,50 |
| P01023 | Alpha-2-macroglobulin (Alpha-2-M) | 56,5 | 83 | 1,47 |
| A2KBC6 | Anti-FactorVIII scFv (Fragment) | 6,5 | 9,5 | 1,46 |
| P01011 | Alpha-1-antichymotrypsin (ACT) (Serpin A3) | 3,5 | 5 | 1,43 |
| P00738 | Haptoglobin | 13,5 | 19 | 1,41 |
| P02743 | Serum amyloid P-component (SAP) | 2,5 | 3,5 | 1,40 |
| P01024 | C3 and PZP-like alpha-2-macroglobulin domain-containing protein 1 | 60,5 | 84,5 | 1,40 |
| A8K008 | cDNA FLJ78387 | 17,5 | 24 | 1,37 |
| A2IPI5 | HRV Fab 026-VL (Fragment) | 3 | 4 | 1,33 |
| Q6UX06 | Olfactomedin-4 (OLM4) (Antiapoptotic protein GW112) | 4,5 | 6 | 1,33 |
| P03952 | Plasma kallikrein (EC 3.4.21.34) (Fletcher factor) | 1,5 | 2 | 1,33 |
| P08567 | Pleckstrin (Platelet 47 kDa protein) (p47) | 1,5 | 2 | 1,33 |
| P07996 | Thrombospondin-1 | 19,5 | 26 | 1,33 |
| P07437 | Tubulin beta chain (Tubulin beta-5 chain) | 4,5 | 6 | 1,33 |
| A2MYD4 | V2-7 protein (Fragment) | 3 | 4 | 1,33 |
| P00488 | Coagulation factor XIII A chain (EC 2.3.2.13) | 3,5 | 4,5 | 1,29 |
| Q71U36 | Tubulin alpha-lA chain (Alpha-tubulin 3) | 3,5 | 4,5 | 1,29 |
| P68363 | Tubulin alpha-1B chain (Alpha-tubulin ubiquitous) | 3,5 | 4,5 | 1,29 |
| Q9BQE3 | Tubulin alpha-1C chain (Alpha-tubulin 6) | 3,5 | 4,5 | 1,29 |
| Q9H4B7 | Tubulin beta-1 chain | 3,5 | 4,5 | 1,29 |
| Q96K68 | cDNA FLJ14473 fis, clone MAMMA1001080 | 12 | 15 | 1,25 |
| P08107 | Heat shock 70 kDa protein 1A/1B | 2 | 2,5 | 1,25 |
| P34931 | Heat shock 70 kDa protein 1-like | 2 | 2,5 | 1,25 |
| P11142 | Heat shock cognate 71 kDa protein | 2 | 2,5 | 1,25 |
| Q6N089 | Putative uncharacterized protein DKFZp686P15220 | 15 | 18,5 | 1,23 |
| P02675 | Fibrinogen beta chain | 11 | 13,5 | 1,23 |
| P00739 | Haptoglobin-related protein | 12 | 14,5 | 1,21 |
| Q86UX7 | Fermitin family homolog 3 (Kindlin-3) | 5 | 6 | 1,20 |
| P02751 | Fibronectin (FN) | 61,5 | 72,5 | 1,18 |
| B7ZLE5 | FN1 protein | 61,5 | 72,5 | 1,18 |
| Q6MZM7 | Putative uncharacterized protein DKFZp686O12165 | 61,5 | 72,5 | 1,18 |
| Q68CX6 | Putative uncharacterized protein DKFZp686O13149 | 61,5 | 72,5 | 1,18 |
| P68032 | Actin, alpha cardiac muscle 1 (Alpha-cardiac actin) | 8,5 | 10 | 1,18 |
| Q562R1 | Beta-actin-like protein 2 (Kappa-actin) | 8,5 | 10 | 1,18 |
| P0C0L4 | Complément C4-A (Acidic complement C4) | 52 | 61 | 1,17 |
| P0C0L5 | Complément C4-B (Basic complement C4) | 52 | 61 | 1,17 |
| B0UZ85 | Complément component 4B (Childo blood group) | 53 | 62 | 1,17 |
| Q6MZQ6 | Putative uncharacterized protein DKFZp686G11190 | 15 | 17,5 | 1,17 |
| P07477 | Trypsin-1 (EC 3.4.21.4) (Serine protease 1) | 3 | 3,5 | 1,17 |
| Q4TZM4 | Hemoglobin beta chain (Fragment) | 6,5 | 7,5 | 1,15 |
| P04004 | Vitronectin (VN) (S-protein) (V75) | 6,5 | 7,5 | 1,15 |
| B2RUT6 | Complément component 4A (Rodgers blood group) | 53,5 | 61,5 | 1,15 |
| P01031 | Complément C5 | 3,5 | 4 | 1,14 |
| Q9UL78 | Myosin-reactive immunoglobulin light chain variable region | 3,5 | 4 | 1,14 |
| Q5NV62 | V3-4 protein (Fragment) | 6 | 7 | 1,17 |
| A6H8M8 | C4A protein (Complement C4 gamma chain) | 53,5 | 61 | 1,14 |
| P02768 | Serum albumin | 51,5 | 58,5 | 1,14 |
| Q7Z351 | Putative uncharacterized protein DKFZp686N02209 | 15 | 17 | 1,13 |
| P02649 | Apolipoprotein E (Apo-E) | 4 | 4,5 | 1,13 |
| P01009 | Alpha-1-antitrypsin (Alpha-1 protease inhibitor) (Serpin A1) | 8,5 | 9,5 | 1,12 |
| Q08380 | Galectin-3-binding protein (Basement membrane autoantigen p105) | 12 | 13 | 1,08 |
| Q6N092 | Putative uncharacterized protein DKFZp686K18196 (Fragment) | 12 | 13 | 1,08 |
| P63261 | Actin, cytoplasmic 2 (Gamma-actin) | 20 | 21,5 | 1,08 |
| P02671 | Fibrinogen alpha chain | 9,5 | 10 | 1,05 |
| P60709 | Actin, cytoplasmic 1 (Beta-actin) | 19,5 | 20,5 | 1,05 |
| A2MYE1 | A30 (Fragment) | 4 | 4 | 1,00 |
| Q96SA9 | Anti-streptococcal/anti-myosin immunoglobulin kappa light chain | 3,5 | 3,5 | 1,00 |
| P06576 | ATP synthase subunit beta, mitochondrial (EC 3.6.3.14) | 2 | 2 | 1,00 |
| O43866 | CD5 antigen-like (CT-2) (IgM-associated peptide) | 15 | 15 | 1,00 |
| P12259 | Coagulation factor V (Activated protein C cofactor) | 1,5 | 1,5 | 1,00 |
| P02747 | Complément C1q subcomponent subunit C | 5,5 | 5,5 | 1,00 |
| P04196 | Histidine-rich glycoprotein (HPRG) | 1,5 | 1,5 | 1,00 |
| P01892 | HLA class I, A-2 alpha chain (MHC class I antigen A*2) | 3 | 3 | 1,00 |
| P30447 | HLA class I histocompatibility antigen, A-23 alpha chain | 3 | 3 | 1,00 |
| F6IQP2 | MHC class I antigen (Fragment) | 3 | 3 | 1,00 |
| F6IR35 | MHC class I antigen (Fragment) | 3 | 3 | 1,00 |
| A2NKM7 | NANUC-2 heavy chain (Fragment) | 3 | 3 | 1,00 |
| P26022 | Pentraxin-related protein PTX3 (Pentaxin-related protein PTX3) | 2 | 2 | 1,00 |
| P02760 | Protein AMBP | 2,5 | 2,5 | 1,00 |
| A2J1M8 | Rheumatoid factor RF-IP12 (Rheumatoid factor RF-IP13) | 3 | 3 | 1,00 |
| A2J1N0 | Rheumatoid factor RF-IP14 (Fragment) | 6 | 6 | 1,00 |
| Q9HCC1 | Single chain Fv (Fragment) | 7,5 | 7,5 | 1,00 |
| H0YLA9 | Uncharacterized protein | 2 | 2 | 1,00 |
| P02774 | Vitamin D-binding protein (DBP) (VDB) | 2 | 2 | 1,00 |
| P02647 | Apolipoprotein A-I (ApoA-I) (Apolipoprotein A1) | 11 | 10,5 | 0,95 |
| P68871 | Hemoglobin subunit beta (Beta-globin) | 8,5 | 8 | 0,94 |
| A2NYQ9 | Anti-folate binding protein (Fragment) | 7 | 6,5 | 0,93 |
| A2JA14 | Anti-mucin1 heavy chain variable region (Fragment) | 6,5 | 6 | 0,92 |
| B6EDE2 | Epididymis luminal protein 180 (Fragment) | 6,5 | 6 | 0,92 |
| Q14477 | Hbbm fused globin protein (Fragment) | 6,5 | 6 | 0,92 |
| Q670S4 | Hemoglobin Lepore-Baltimore (Fragment) | 6,5 | 6 | 0,92 |
| P02042 | Hemoglobin subunit delta (Delta-globin) | 6,5 | 6 | 0,92 |
| Q9UL90 | Myosin-reactive immunoglobulin heavy chain variable region | 6,5 | 6 | 0,92 |
| A2NZ55 | Variable immnoglobulin anti-estradiol heavy chain | 6,5 | 6 | 0,92 |
| Q15485 | Ficolin-2 (37 kDa elastin-binding protein) | 5,5 | 5 | 0,91 |
| Q9UL88 | Myosin-reactive immunoglobulin heavy chain variable region | 5 | 4,5 | 0,90 |
| Q6ZVX0 | cDNA FLJ41981 fis, clone SMINT2011888 | 9,5 | 8,5 | 0,89 |
| Q7Z374 | Putative uncharacterized protein DKFZp686C02218 | 9,5 | 8,5 | 0,89 |
| Q6MZX9 | Putative uncharacterized protein DKFZp686M08189 | 9,5 | 8,5 | 0,89 |
| P02679 | Fibrinogen gamma chain | 12,5 | 11 | 0,88 |
| Q6P5S8 | antioxidant activity; very-low-density lipoprotein particle remodeling | 16 | 14 | 0,88 |
| P27105 | Erythrocyte band 7 integral membrane protein (Stomatin) | 4 | 3,5 | 0,88 |
| P69905 | Hemoglobin subunit alpha (Alpha-globin) | 4 | 3,5 | 0,88 |
| Q6GMX0 | Putative uncharacterized protein | 16 | 14 | 0,88 |
| P02792 | Ferritin light chain (Ferritin L subunit) | 3,5 | 3 | 0,86 |
| Q9UL71 | Myosin-reactive immunoglobulin heavy chain variable region | 7 | 6 | 0,86 |
| Q07954 | Prolow-density lipoprotein receptor-related protein 1 (LRP-1) | 7 | 6 | 0,86 |
| A1A508 | PRSS3 protein | 3,5 | 3 | 0,86 |
| Q6P5S3 | Putative uncharacterized protein | 7 | 6 | 0,86 |
| Q65ZC9 | Single-chain Fv (Fragment) | 7 | 6 | 0,86 |
| Q5CZ94 | Putative uncharacterized protein DKFZp781M0386 | 6,5 | 5,5 | 0,85 |
| P19823 | Inter-alpha-trypsin inhibitor heavy chain H2 (ITI-HC2) | 9,5 | 8 | 0,84 |
| Q16195 | Keratin (Fragment) | 9,5 | 8 | 0,84 |
| P30464 | HLA class I, B-15 alpha chain (MHC class I antigen B*15) | 3 | 2,5 | 0,83 |
| P03989 | HLA class I, B-27 alpha chain (MHC class I antigen B*27) | 3 | 2,5 | 0,83 |
| Q9UL83 | Myosin-reactive immunoglobulin light chain variable region | 3 | 2,5 | 0,83 |
| P35579 | Myosin-9 (Myosin heavy chain 9) | 8,5 | 7 | 0,82 |
| A0A5E4 | Putative uncharacterized protein | 8,5 | 7 | 0,82 |
| P10909 | Clusterin (Apolipoprotein J) (Apo-J) | 5,5 | 4,5 | 0,82 |
| Q5EFE6 | Anti-RhD monoclonal T125 kappa light chain | 16 | 13 | 0,81 |
| Q86TT1 | Full-length cDNA clone CS0DD006YL02 of Neuroblastoma | 28 | 22,5 | 0,80 |
| O14791 | Apolipoprotein L1 (ApoL-I) | 2,5 | 2 | 0,80 |
| A2NB45 | Cold agglutinin FS-1 L-chain (Fragment) | 2,5 | 2 | 0,80 |
| A2NB44 | Cold agglutinin FS-2 H-chain (Fragment) | 2,5 | 2 | 0,80 |
| B1N7B8 | Cryocrystalglobulin CC1 kappa light chain variable region | 2,5 | 2 | 0,80 |
| Q86SX2 | Full-length cDNA clone CS0DL004YM19 of B cells | 2,5 | 2 | 0,80 |
| Q7Z3Y6 | Rearranged VH4-34 V gene segment (Fragment) | 2,5 | 2 | 0,80 |
| A0N5G5 | Rheumatoid factor D5 light chain (Fragment) | 2,5 | 2 | 0,80 |
| H0YK52 | Uncharacterized protein | 2,5 | 2 | 0,80 |
| Q14624 | Inter-alpha-trypsin inhibitor heavy chain H4 (ITI-HC4) | 16 | 12,5 | 0,78 |
| P31946 | 14-3-3 protein beta/alpha (KCIP-1) | 4,5 | 3,5 | 0,78 |
| P62258 | 14-3-3 protein epsilon (14-3-3E) | 4,5 | 3,5 | 0,78 |
| Q04917 | 14-3-3 protein eta (Protein AS1) | 4,5 | 3,5 | 0,78 |
| P61981 | 14-3-3 protein gamma (KCIP-1) | 4,5 | 3,5 | 0,78 |
| P27348 | 14-3-3 protein theta (Protein HS1) | 4,5 | 3,5 | 0,78 |
| A2NUT2 | Lambda-chain (AA -20 to 215) | 9 | 7 | 0,78 |
| P02776 | Platelet factor 4 (PF-4) | 4 | 3 | 0,75 |
| P10720 | Platelet factor 4 variant (CXCL4L1) | 4 | 3 | 0,75 |
| P02746 | Complément C1q subcomponent subunit B | 9,5 | 7 | 0,74 |
| P48740 | Mannan-binding lectin serine protease 1 (EC 3.4.21.-) | 7,5 | 5,5 | 0,73 |
| Q96JD0 | Amyloid lambda 6 light chain variable region SAR (Fragment) | 9 | 6,5 | 0,72 |
| Q6GMV8 | Putative uncharacterized protein | 9 | 6,5 | 0,72 |
| Q8NEJ1 | Putative uncharacterized protein | 9 | 6,5 | 0,72 |
| A0N5G3 | Rheumatoid factor G9 light chain (Fragment) | 9 | 6,5 | 0,72 |
| P02745 | Complément C1q subcomponent subunit A | 3,5 | 2,5 | 0,71 |
| P60660 | Myosin light polypeptide 6 (MLC-3) | 3,5 | 2,5 | 0,71 |
| Q6MZU6 | Putative uncharacterized protein DKFZp686C15213 | 17,5 | 12,5 | 0,71 |
| Q6N093 | Putative uncharacterized protein DKFZp686I04196 (Fragment) | 17,5 | 12,5 | 0,71 |
| P61224 | Ras-related protein Rap-1b (GTP-binding protein smg p21B) | 3 | 2 | 0, 67 |
| P19105 | Myosin regulatory light chain 12A (MLC-2B) | 4 | 2,5 | 0,63 |
| P05387 | 60S acidic ribosomal protein P2 (Renal carcinoma antigen NY-REN-44) | 2,5 | 1,5 | 0,60 |
| P19338 | Nucleolin (Protein C23) | 10 | 6 | 0,60 |
| O75636 | Ficolin-3 (Collagen/fibrinogen domain-containing lectin 3 p35) | 3,5 | 2 | 0,57 |
| F8VWA4 | Uncharacterized protein | 3,5 | 2 | 0,57 |
| B7Z539 | cDNA FLJ56954 | 8 | 4,5 | 0,56 |
| Q96CX2 | BTB/POZ domain-containing protein KCTD12 (Pfetin) | 4,5 | 2,5 | 0,56 |
| P02730 | Band 3 anion transport protein(AE 1) | 17,5 | 9,5 | 0,54 |
| P23528 | Cofilin-1 (p18) | 3 | 1,5 | 0,50 |
| P06748 | Nucleophosmin (NPM) | 3 | 1,5 | 0,50 |
| Q6N091 | Putative uncharacterized protein DKFZp686C02220 | 7 | 3,5 | 0,50 |
| Q6N041 | Putative uncharacterized protein DKFZp686O16217 (Fragment) | 7 | 3,5 | 0,50 |
| P11166 | Solute carrier family 2, facilitated glucose transporter member 1 | 2 | 1 | 0,50 |
| P04275 | von Willebrand antigen 2 | 61,5 | 28,5 | 0,46 |
| P62805 | Histone H4 | 2,5 | 1 | 0,40 |
| Q13201 | Multimerin-1 (EMILIN-4) | 11 | 4 | 0,36 |
| B7Z1F8 | cDNA FLJ53025, highly similar to Complément C4-B | 52 | 16 | 0,31 |
| P04114 | Apolipoprotein B-100 (Apo B-100) | 25 | 7,5 | 0,30 |
| Q8TCG3 | TPMsk3 (Fragment) | 5 | 1,5 | 0,30 |
| P16452 | Erythrocyte membrane protein band 4.2 (P4.2) | 5,5 | 1 | 0,18 |
| P11277 | Spectrin beta chain, erythrocyte (Beta-I spectrin) | 23 | 3 | 0,13 |
| P02549 | Spectrin alpha chain, erythrocyte (Erythroid alpha-spectrin) | 33 | 1,5 | 0,05 |

**Tableau 2b**

| **Numéro d'accession** | **Nom protéine** | **Nombre moyen de peptides DS** |
|---|---|---|
| P00751 | Complément factor B (C3/C5 convertase) | 4.5 |
| P00752 | Complément component C8 alpha chain | 2 |
| P00753 | Peroxiredoxin-1 (Natural killer cell-enhancing factor A) | 2 |
| P00754 | Moesin (Membrane-organizing extension spike protein) | 2 |
| P00755 | Chloride intracellular channel protein 1 | 2 |
| P00756 | Ferritin heavy chain (Ferritin H subunit) | 2 |

### Exemple 4 : ELISA de confirmation

### Méthode :

Afin de confirmer les résultats de spectrométrie de masse, des ELISA quantitatifs spécifiques ont été effectués en duplicate sur des plasmas individuels. Les protéines sélectionnées et testées, parmi celles identifiées dans les tableaux 2a/2b, sont celles qui présentaient un rapport dengue sévère (DS)/dengue classique (DF) supérieur ou égal à 1,33 et inférieur ou égal à 0,75 avec un nombre moyen de peptides supérieur à 1 pour chaque échantillon et un lien potentiel avec la pathogénie de la dengue. Ce premier criblage a permis de ne tester que les protéines présentant le plus d'intérêt. Suivant ces critères, les protéines suivantes ont été sélectionnées :
- la céruloplasmine,
- la protéine S,
- le complément facteur properdine,
- l'antithrombine III,
- le composant sécrétoire p85,
- la protéine du complément C1r,
- la protéine du complément C1s,
- l'angiotensine,
- le facteur II,
- le CFB,
- l'anti-Facteur VIII,
- le sérum amyloide P-composant,
- l'olfactomedine 4 (OLFM4),
- la trombospondine,
- le facteur plaquettaire 4 (PF4),
- la protéine du complément C1q,
- la moésine, et
- la protéine du complément C8.

La multimerine-1, l'apolipoproteine B-100 et le facteur von Willebrand ont également été testées.

Il convient de noter que ces protéines sont majoritairement des éléments de la voie de coagulation ou de la cascade du complément.

Ces ELISA ont été effectués grâce à des kits commerciaux (USCN, China), en suivant les instructions des fournisseurs. Les analyses statistiques (Test de Man-Whitney et courbe de ROC/AUC) ont été effectuées à l'aide du logiciel GraphPad Prism V4.03.

Chaque marqueur-candidat a été testé sur des échantillons de plasma individuels. Ces échantillons sont des échantillons de plasma prélevés durant la phase fébrile aiguë de la maladie (phase virémique), ces échantillons provenant soit de patients étant restés avec une dengue classique DF, sans évoluer vers une dengue sévère, soit de patients ayant évolué vers une dengue sévère DS. Tous les patients avaient une dengue secondaire. Seuls les sérotypes 1, 2 et 3 étaient représentés (pas de sérotype 4). Ces prélèvements étaient originaires de *l'Universidad Industrial de Santander* (Bucaramanga, Colombie) [5] ou de l'Institut Pasteur du Cambodge (Phnom-Penh). Ces derniers faisaient partie d'une étude prospective menée en accord avec le comité éthique local. Les caractéristiques des deux sources de prélèvements sont détaillées dans les tableaux 3 et 4 qui suivent. Les échantillons ont été collectés après apparition des symptômes.

**Tableau 3**

| | **DF (n=15)** | **DS (n=15)** | **Valeur de p** |
|---|---|---|---|
| **Age moyen +-SD (an)** | 29.3+-0.52 | 28.3+-0.5 | ns |
| **% male** | 60% | 53% | ns |
| **Prélèvement (jour)** | 3.06 | 2.37 | ns |
| **Poids moyen+-SD (Kg)** | 60.2 +-8.6 | 50.3+-15.6 | ns |
| **Cholestérol total (g/L)** | 1.44+-5.4 | 1.29+-10.1 | 0.03 |
| **AST (U/L)** | 90.2+-9.75 | 142.7+-29.8 | 0.001 |
| **ALT (U/L)** | 69.7+-11.4 | 90.8+-24.5 | ns |
| **Test tourniquet positif** | 8/15 (60%) | 10/15 (66%) | ns |
| **Charge virale (copie/mL)** | 4.05 10⁶+-3.5 | 4.1 10⁷+-5.22 | 0.006 |
| **Serotype** | DV2-DV3 | DV2-DV3 | |
| **Dengue secondaire** | oui | oui | |
| **Comorbidité** | non | non | |

**Tableau 4**

| | **DF (n=23)** | **DS (n=26)** | **Valeur de p** |
|---|---|---|---|
| **Age moyen+-SD age (an)** | 8.6+-0.38 | 7.3+-0.45 | ns |
| **% male** | 56% | 35.6% | ns |
| **Prélèvement (jour)** | 2.6 | 3.2 | ns |
| **Poids moyen+-SD (Kg)** | 20.48+-0.93 | 18.86+-0.98 | ns |
| **Cholestérol total (mmol/L)** | 3.32+-0.14 | 2.37+-0.12 | <0.0001 |
| **HDL (mmol/L)** | 0.82+-0.07 | 0.31+-0.02 | <0.0001 |
| **Triglycérides (g/L)** | 1.37+-0.17 | 2.8+-0.2 | <0.0001 |
| **AST (IU/L)** | 133.3+-23.18 | 302.5+-50.7 | 0.0043 |
| **ALT (IU/L)** | 66.9+-13.4 | 106.3+-21.2 | ns |
| **Charge virale (copie/mL)** | 7.1 10⁸+-6 | 2.06 10⁸+-2 | ns |
| **Test tourniquet Positif** | 19/23 (82%) | 18/26 (82.6%) | ns |
| **HGB** | 11.63+-0.15 | 13.3+-0.29 | <0.0001 |
| **Hématocrite** | 38.23+-0.57 | 41.62+-0.8 | 0.0013 |
| **Hépatomégalie (Ultrasons)** | 8/12 (66%) | 16/26 (61.5%) | ns |
| **Serotype** | DV1 | DV1 | - |
| **Dengue Secondaire** | oui | oui | - |
| **Comorbidité** | non | non | - |

| | | | |
|---|---|---|---|
| SD : déviation standard ns : valeur de p non significative | | | |

### Résultats :

Pour la majorité des marqueurs testés en ELISA, aucune différence de concentration plasmatique n'a été constatée entre les plasmas DF et DS, qu'ils soient colombiens ou cambodgiens (p>0.1).

Par contre, pour deux marqueurs les résultats permettent de distinguer clairement les patients qui ont ensuite développé une dengue sévère (DS) de ceux restant uniquement avec une dengue classique sans évoluer vers une dengue sévère. Le premier marqueur est PF4 (Platelet factor 4 ou facteur plaquettaire 4).

Pour les échantillons colombiens une différence de concentration plasmatique en faveur des échantillons DF est observée (p<0.001) (figure 2). L'AUC est de 0.88 (95%IC : 0.7305-1).

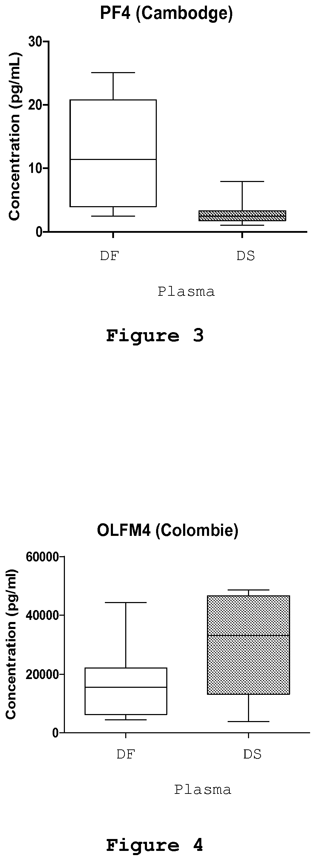

Ceci est confirmé pour les échantillons cambodgiens pour lesquels on a une différence de concentration plasmatique significative en faveur des échantillons DF (p< 0.0001) (figure 3). L'AUC est de 0.94 (95%IC=0.87-1). La courbe de ROC a permis de déterminer quelle était la meilleure spécificité pour une sensibilité voisine de 100%. Les résultats sont résumés dans le tableau 5 ci-dessous: pour ce marqueur, pour une sensibilité de 95%, on atteint une spécificité de près de 78%.

**Tableau 5**

| | **Pour une sensibilité de :** | **La meilleure spécificité est :** |
|---|---|---|
| **PF4** | 100 | 61 |
| | 95 | 77.9 |

- Le deuxième marqueur est OLFM4 (Olfactomedine 4)

Pour les échantillons colombiens, la concentration plasmatique du marqueur est supérieure dans les échantillons DS par rapport aux échantillons DF (p=0.07 ; Cf. Figure 4).

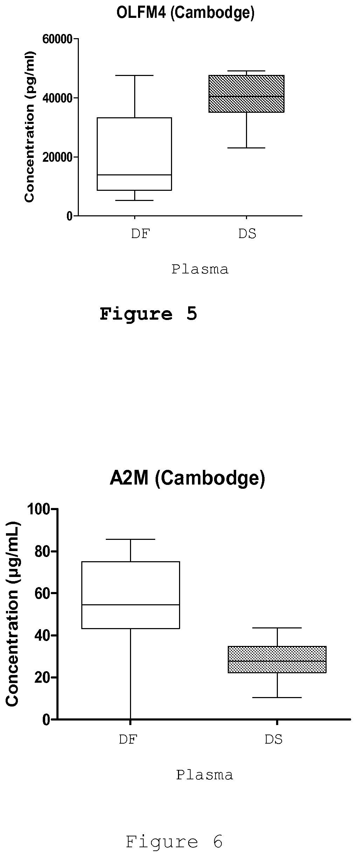

Ceci est confirmé sur les échantillons cambodgiens avec une différence de concentration extrêmement significative (p<0.0003) et une médiane plus de 2 fois supérieure pour les échantillons DS comparés aux échantillons DF (Figure 5). L'AUC est de 0.858 (95%IC= 0.7307-0.985). La courbe de ROC a permis de déterminer quelle était la meilleure spécificité pour une sensibilité voisine de 100%. Les résultats sont résumés dans le tableau 6 : pour ce marqueur, pour une sensibilité voisine de 95%, on atteint une spécificité supérieure à 72%.

**Tableau 6**

| | **Pour une sensibilité de :** | **La meilleure spécificité est :** |
|---|---|---|
| **OLFM4** | 100 | 61 |
| | 94.4 | 72.2 |

En parallèle, un autre marqueur, l'alpha-2 macroglobuline (A2M), a été identifié à partir d'échantillons de plasmas cambodgiens non purifiés, par une méthode de protéomique différentielle de type SILAC (*Stable Isotope Labelling by Aminoacids in cell Culture*) [6]. L'identification de ce troisième marqueur est décrit dans les exemples qui suivent.

### Exemple 5 : Caractérisation des échantillons

La composition de chaque groupe ou pool de plasma utilisé dans cette expérience est résumée dans le tableau 7. Tous les plasmas cambodgiens sélectionnés pour la constitution des pools ont été prélevés durant la phase fébrile aiguë de la maladie, avant la phase critique, sur des malades ayant une infection secondaire. Le sérotype concerné était le sérotype 1. Tous les patients DS étaient hospitalisés et présentaient des signes d'hémorragies. Aucune comorbidité n'a été reportée. Tous les plasmas ont été contrôlés NS1-positif (Kit Platelia Dengue Biorad) et la charge virale a également été vérifiée en Q-RTPCR par un kit commercial suivant les instructions du fournisseur. Les pools constitués correspondent à un volume final d'environ 2 mL de plasma. Les groupes de plasma sont préalablement inactivés à la chaleur (56°C/20 minutes) puis préclarifiés par une centrifugation de 5 min à 1000g et à 4°C, afin d'enlever les impuretés présentes dans l'échantillon.

**Tableau 7**

| | DF (n=6) | DS (n=6) |
|---|---|---|
| Sérotype | DV1 | DV1 |
| Dengue secondaire | OUI | OUI |
| Age en année (moyenne) | 6-12 (8.6) | 6-8 (7) |
| Male/femelle | 2/4 | 2/4 |
| NS1 positif / virus positif | OUI/OUI | OUI /OUI |
| Jour moyen de prélèvement | 4 | 3.85 |
| Grade sévérité | 0-1 | 3-4 |
| Comorbidité | NON | NON |

### Exemple 6 : Analyse en protéomique différentielle

La méthode utilisée est une méthode protéomique semi-quantitative de type SILAC (*Stable Isotope Labelling by Aminoacids in cell Culture*) [6] développée par la société Pronota (Gand, Belgique) en utilisant la plate-forme MASStermind^{tm} et exécuté sur les groupes de plasma dengue classique DF ou dengue sévère DS. Chaque groupe est constitué d'un mélange de 6 échantillons, comme détaillé dans l'exemple 5.

Ces mélanges de plasma ont préalablement été déplétés des 14 protéines plasmatiques les plus abondantes par chromatographie d'affinité. La quantité de protéines récupérées au final a été obtenue par un test colorimétrique à base d'acide bicinchoninique (BCA assay ThermoFicher Scientific). Inc., USA).

L'étude MASStermind^{tm} a comparé chaque échantillon à un échantillon de référence qui regroupe tous les échantillons. Cette méthode fournit des informations sur les niveaux relatifs et l'absence ou la présence de peptides / protéines dans des échantillons dengue sévère DS en comparaison des échantillons dengue classique DF. L'analyse différentielle est réalisée en mélangeant les échantillons marqués par différents isotopes et en analysant par spectrométrie de masse chaque pic apparié. Le marqueur isotopique est introduit par hydrolyse trypsique qui incorpore 2 atomes ¹⁸O (marquage « lourd ») sur l'arginine C-terminale d'un peptide, ce qui entraîne une différence de masse 4 daltons avec le même peptide marqué ¹⁶O (« marquage léger ») . L'échantillon de référence est étiqueté avec ¹⁶O alors que les échantillons individuels sont étiquetés avec ¹⁸O. Les données MS / MS sont ensuite soumises au logiciel MASCOT pour l'identifications des peptides et des protéines dans chaque échantillons.

Suite à l'analyse MS / MS, plus de 250 protéines quantifiables ont pu être identifiées, dont 10 protéines qui ont au moins 1 peptide trouvé différentiel. Pour chaque protéine identifiée le ratio DS / DF est calculé comme la moyenne pondérée des coefficients de tous les peptides identifiés pour la protéine donnée. Globalement, les résultats ont montré une grande similitude entre les deux protéomes et seulement quelques protéines ont été trouvées être exprimées de manière différentielle. Pour trois de ces dix protéines, les peptides identifiés sont systématiquement exprimés de manière différentielle et présentent un rapport DS/DF moyen qui s'écarte de 1 (**cf. Tableau 8**). Les trois protéines identifiées sont : l'alpha-2 macroglobuline (A2M), le complément C3f et l'héparine cofacteur-2 . Ces protéines sont majoritairement des éléments de la voie de coagulation ou de la cascade du complément.

**Tableau 8**

| Protéines | Numéro d'accession | Rapport DS/DF | 95% IC sur moyenne des ratios |
|---|---|---|---|
| Alpha 2 macroglobuline | A2MG_HUMAN | 0.33 | 0.25-0.44 |
| Complément C3f | CO3_HUMAN | 0.89 | 0.68-1.17 |
| Héparine cofacteur 2 | HEP2_HUMAN | 2.25 | 1.71-2.95 |

### Exemple 7 : ELISA de confirmation

### Méthode :

Afin de confirmer les résultats de spectrométrie de masse, des ELISA quantitatifs spécifiques ont été effectués en duplicate sur des plasmas individuels. Les protéines testées sont celles identifiées dans l'exemple 6 : l'alpha-2 Macroglobuline (A2M), La protéine C3 du complément, l'héparine co-facteur 2.

Ces ELISA ont été effectués grâce à des kits commerciaux (USCN, China), en suivant les instructions du fournisseur. Les analyses statistiques (Test de Man-Whitney et courbe de ROC/AUC) ont été effectuées à l'aide du logiciel GraphPad Prism V4.03.

Chaque marqueur-candidat a été testé sur des échantillons individuels. Ces échantillons étaient des plasmas de patients prélevés durant la phase fébrile aiguë de la maladie (phase virémique). Le suivi clinique des patients a montré que certains étaient finalement restés avec une dengue classique DF sans évoluer vers une dengue sévère, alors que d'autres avaient évolué vers une dengue sévère DS. Tous les patients avaient une dengue secondaire. Seul le sérotype 1 était représenté. Ces prélèvements étaient originaire de l'Institut Pasteur du Cambodge (Phnom-Penh) et faisaient partie d'une étude prospective menée en accord avec le comité éthique local. Les caractéristiques de la source de prélèvements sont détaillées dans le tableau 9.

**Tableau 9**

| | DF (n=23) | DS (n=26) | Valeur de p |
|---|---|---|---|
| Age moyen+-SD age (an) | 8.6+-0.38 | 7.3+-0.45 | ns |
| % male | 56% | 35.6% | ns |
| Prélèvement (jour) | 2.6 | 3.2 | ns |
| Poids moyen+-SD (Kg) | 20.48+-0.93 | 18.86+-0.98 | ns |
| Cholestérol total (mmol/L) | 3.32+-0.14 | 2.37+-0.12 | <0.0001 |
| HDL (mmol/L) | 0.82+-0.07 | 0.31+-0.02 | <0.0001 |
| Triglycérides (g/L) | 1.37+-0.17 | 2.8+-0.2 | <0.0001 |
| AST (IU/L) | 133.3+-23.18 | 302.5+-50.7 | 0.0043 |
| ALT (IU/L) | 66.9+-13.4 | 106.3+-21.2 | ns |
| Charge virale (copie/mL) | 7.1 10⁸+-6 | 2.06 10⁸+-2 | ns |
| Test tourniquet Positif | 19/23 (82%) | 18/26 (82.6%) | ns |
| HGB | 11.63+-0.15 | 13.3+-0.29 | <0.0001 |
| Hématocrite | 38.23+-0.57 | 41.62+-0.8 | 0.0013 |
| Hépatomégalie (Ultrasons) | 8/12 (66%) | 16/26 (61.5%) | ns |
| Sérotype | DV1 | DV1 | - |
| Dengue Secondaire | oui | oui | - |
| Comorbidité | non | non | - |

| | | | |
|---|---|---|---|
| HBG : hemoglobine plasmatique SD : déviation standard ns : valeur de p non significative | | | |

### Résultats :

Pour la majorité des marqueurs testés en ELISA (le complément C3f et l'héparine cofacteur 2), aucune différence de concentration plasmatique n'a été constatée entre les plasma DF et DS (p>0.1).

Cependant, pour les échantillons cambodgiens testés en ELISA pour A2M, la différence de concentration était significative (p<0.0004) avec une médiane environ 2 fois supérieure pour les échantillons DF comparés aux échantillons DS (Figure **6**). L'AUC était de 0.89 (95%IC= 0.75-1).

Pour A2M, la courbe de ROC a permis de déterminer quelle était la meilleure spécificité pour une sensibilité voisine de 100%. Les résultats sont résumés dans le tableau **10 :** pour une sensibilité voisine de 94%, on atteint une spécificité supérieure à 83%.

**Tableau 10**

| | Pour une sensibilité de : | La meilleure spécificité est : |
|---|---|---|
| A2M | 100 | 72 |
| | 93.8 | 83.3 |

### REFERENCES BIBLIOGRAPHIQUES

1. SB Halstead . The lancet 2007; 370: 1644-52
2. AS Leong et al. Semin.Diagn.Pathol. 2007; 24(4):227-236
3. K. Clyde et al. J. Virol. 2006; 23: 11418-11431
4. Fields Virology, Fifth édition, Knipe DM ed., LWW
5. Villar-Centeno LA et al. Am. J.Trp.Med.Hyg. 2008; 78 : 370-374
6. R. Drissi et al. FEBS J. 2013
7.Srichaikul et al. Southeast Asian J Trop Med Public Health. 1989

## Revendications

1. Procédé pour déterminer, *in vitro*, dans un échantillon sanguin, la probabilité pour un patient d'évoluer vers une dengue sévère, dans lequel :
a) on détermine la quantité d'au moins un marqueur qui est le facteur plaquettaire 4 dans ledit échantillon sanguin,
b)on compare la quantité du facteur plaquettaire 4 déterminée dans l'étape a) avec une quantité de référence dudit marqueur obtenue à partir d'un groupe d'individus qui ont été diagnostiqués comme étant atteints d'une dengue non sévère,
dans lequel, si la quantité du facteur plaquettaire 4 déterminée dans l'étape a) est inférieure à la quantité de référence établie dans l'étape b), on détermine que le patient va évoluer vers une dengue sévère.

2. Procédé selon la revendication 1, dans lequel dans l'étape a) on détermine la quantité d'au moins un autre marqueur choisi parmi l'olfactomedine-4 et l'α2-macroglobuline dans l'échantillon sanguin, et dans l'étape b) on compare la quantité dudit autre marqueur de l'étape a) avec une quantité de référence dudit autre marqueur obtenue à partir d'un groupe d'individus qui ont été diagnostiqués comme étant atteints d'une dengue non sévère, et si la quantité dudit au moins autre marqueur déterminée dans l'étape a) est supérieure pour l'olfactomedine 4, ou inférieure pour l'α2-macroglobuline, à la quantité de référence établie dans l'étape b), on détermine que le patient va évoluer vers une dengue sévère.

3. Utilisation du facteur plaquettaire 4 comme marqueur protéique pour déterminer, *in vitro*, dans un échantillon sanguin, la probabilité pour un patient d'évoluer vers une dengue sévère.

4. Utilisation selon la revendication 3, comprenant également l'utilisation de l'α2-macroglobuline comme marqueur protéique pour déterminer, *in vitro*, dans un échantillon sanguin, la probabilité pour un patient d'évoluer vers une dengue sévère.

5. Utilisation d'un kit pour déterminer, in vitro, la probabilité pour un patient d'évoluer vers une dengue sévère, le kit comprenant un partenaire de liaison du facteur plaquettaire 4 et un partenaire de liaison de la protéine NS1 du virus de la dengue, lesdits partenaires de liaison étant choisis parmi un récepteur, un anticorps, un fragment d'anticorps, un analogue d'anticorps ou tout autre ligand.

6. Kit pour déterminer, *in vitro*, la probabilité pour un patient d'évoluer vers une dengue sévère comprenant un partenaire de liaison du facteur plaquettaire 4 et un partenaire de liaison de l'α2-macroglobuline, lesdits partenaires de liaison étant choisis parmi un récepteur, un anticorps, un fragment d'anticorps, un analogue d'anticorps ou tout autre ligand.

7. Kit selon la revendication 6, comprenant également un partenaire de liaison de la protéine NS1 du virus de la dengue.

8. Kit pour le pronostic, *in vitro*, d'une dengue sévère comprenant un partenaire de liaison du facteur plaquettaire 4, un partenaire de liaison de l'olfactomedine 4, un partenaire de liaison de l'α2-macroglobuline et un partenaire de liaison de la protéine NS1 du virus de la dengue, lesdits partenaires de liaison étant choisis parmi un récepteur, un anticorps, un fragment d'anticorps, un analogue d'anticorps ou tout autre ligand.

## Patentansprüche

1. Verfahren zur in-vitro-Bestimmung, in einer Blutprobe, der Wahrscheinlichkeit eines Patienten, einen schweren Fall von Dengue zu entwickeln, wobei:
a) die Menge von mindestens einem Marker, bei dem es sich um Plättchenfaktor 4 handelt, in der Blutprobe bestimmt wird,
b) die in Schritt a) bestimmte Menge von Plättchenfaktor 4 mit einer Bezugsmenge des Markers verglichen wird, die aus einer Gruppe von Individuen erhalten wird, bei denen ein nicht schwerer Fall von Dengue diagnostiziert wurde,
wobei, wenn die in Schritt a) bestimmte Menge von Plättchenfaktor 4 geringer ist als die in Schritt b) ermittelte Bezugsmenge, festgestellt wird, dass der Patient einen schweren Fall von Dengue entwickelt.

2. Verfahren nach Anspruch 1, wobei in Schritt a) die Menge von mindestens einem weiteren Marker, der aus dem Olfactomedin 4 und α2-Makroglobulin ausgewählt wird, in der Blutprobe bestimmt wird, und in Schritt b) die Menge des weiteren Markers aus Schritt a) mit einer Bezugsmenge des weiteren Markers verglichen wird, die aus einer Gruppe von Individuen erhalten wird, bei denen ein nicht schwerer Fall von Dengue diagnostiziert wurde, und wenn die in Schritt a) bestimmte Menge des mindestens einen weiteren Markers größer für das Olfactomedin 4, oder geringer für das α2-Makroglobulin, als die in Schritt b) ermittelte Bezugsmenge ist, festgestellt wird, dass der Patient einen schweren Fall von Dengue entwickelt.

3. Verwendung von Plättchenfaktor 4 als Proteinmarker zur in-vitro-Bestimmung, in einer Blutprobe, der Wahrscheinlichkeit eines Patienten, einen schweren Fall von Dengue zu entwickeln.

4. Verwendung nach Anspruch 3, die ebenfalls die Verwendung des α2-Makroglobulins als Proteinmarker zur in-vitro-Bestimmung, in einer Blutprobe, der Wahrscheinlichkeit eines Patienten umfasst, einen schweren Fall von Dengue zu entwickeln.

5. Verwendung eines Kits zur in-vitro-Bestimmung der Wahrscheinlichkeit eines Patienten, einen schweren Fall von Dengue zu entwickeln, wobei das Kit einen Bindungspartner für Plättchenfaktor 4 und einen Bindungspartner des NS1-Proteins des Dengue-Virus umfasst, wobei die Bindungspartner aus einem Rezeptor, einem Antikörper, einem Antikörperfragment, einem Antikörperanalogon oder jedem beliebigen anderen Liganden ausgewählt sind.

6. Kit zur in-vitro-Bestimmung der Wahrscheinlichkeit eines Patienten, einen schweren Fall von Dengue zu entwickeln, das einen Bindungspartner für Plättchenfaktor 4 und einen Bindungspartner des α2-Makroglobulins umfasst, wobei die Bindungspartner aus einem Rezeptor, einem Antikörper, einem Antikörperfragment, einem Antikörperanalogon oder jedem beliebigen anderen Liganden ausgewählt sind.

7. Kit nach Anspruch 6, das zudem einen Bindungspartner des NS1-Proteins des Dengue-Virus umfasst.

8. Kit zur in-vitro-Prognose eines schweren Falls von Dengue, das einen Bindungspartner für Plättchenfaktor 4, einen Bindungspartner für Olfactomedin 4, einen Bindungspartner für α2-Makroglobulin und einen Bindungspartner des NS1-Proteins des Dengue-Virus umfasst, wobei die Bindungspartner aus einem Rezeptor, einem Antikörper, einem Antikörperfragment, einem Antikörperanalogon oder jedem beliebigen anderen Liganden ausgewählt sind.

## Claims

1. A method for determining, in *vitro*, the probability of a patient developing severe dengue, based on a blood sample, wherein:
a) the quantity in said blood sample of at least one marker, which is platelet factor 4, is determined,
b) the quantity of platelet factor 4 determined in step a) is compared with a reference quantity of said marker obtained from a group of individuals who have been diagnosed with non-severe dengue,
wherein, if the quantity of platelet factor 4 determined in step a) is less than the reference quantity established in step b), it is determined that the patient will develop severe dengue.

2. The method as claimed in claim 1, wherein, in step a), the quantity of at least one other marker chosen from olfactomedin 4 and α2-macroglobulin is determined in the blood sample and, in step b), the quantity of said other marker of step a) is compared with a reference quantity of said other marker obtained from a group of individuals who have been diagnosed with non-severe dengue and, if the quantity of said at least one other marker determined in step a) is greater for olfactomedin 4, or less for α2-macroglobulin, than the reference quantity established in step b), it is determined that the patient will develop severe dengue.

3. The use of platelet factor 4 as protein marker for determining, in *vitro*, the probability of a patient developing severe dengue, based on a blood sample.

4. The use as claimed in claim 3, also comprising the use of α2-macroglobulin as protein marker for determining, *in vitro*, the probability of a patient developing severe dengue, based on a blood sample.

5. The use of a kit for determining, *in vitro*, the probability of a patient developing severe dengue, the kit comprising a binding partner for platelet factor 4 and a binding partner for the dengue virus NS1 protein, said binding partners being chosen from a receptor, an antibody, an antibody fragment, an antibody analog or any other ligand.

6. A kit for determining, *in vitro*, the probability of a patient developing severe dengue, comprising a binding partner for platelet factor 4 and a binding partner for α2-macroglobulin, said binding partners being chosen from a receptor, an antibody, an antibody fragment, an antibody analog or any other ligand.

7. The kit as claimed in claim 6, also comprising a binding partner for the dengue virus NS1 protein.

8. A kit for the prediction, *in vitro*, of severe dengue comprising a binding partner for platelet factor 4, a binding partner for olfactomedin 4, a binding partner for α2-macroglobulin and a binding partner for the dengue virus NS1 protein, said binding partners being chosen from a receptor, an antibody, an antibody fragment, an antibody analog or any other ligand.
